# EUROPEAN PATENT SPECIFICATION

(11) **EP 1 572 223 B1**
(45) Date of publication and mention of the grant of the patent: **22.06.2011**
(21) Application number: 03814818.5
(22) Date of filing: 16.12.2003
(51) Int. Cl.: A61K 8/97, A61Q 19/02, A61Q 1/04, A61Q 19/08

(54) **USE OF ACTIVE EXTRACTS TO IMPROVE THE APPEARANCE OF SKIN, LIPS, HAIR AND/OR NAILS**
VERWENDUNG VON WIRKEXTRAKTEN ZUR VERBESSERUNG DES ERSCHEINUNGSBILDS VON HAUT, LIPPEN, HAAR UND/ODER NÄGELN
UTILISATION D'EXTRAITS ACTIFS EN VUE D'AMELIORER L'APPARENCE DE LA PEAU, DES LEVRES, DES CHEVEUX ET/OU DES ONGLES

(30) Priority: 17.12.2002 US 321706
(43) Date of publication of application: 14.09.2005
(73) Proprietor: AVON PRODUCTS, INC., New York, NY 10020-1196 (US)
(72) Inventor: MAHALINGAM, Harish, Ledgewood, NJ 07852 (US); JONES, Brian, Flower Mound, Texas 75028 (US); MCCAIN, Nicole, Bronx, NY 10461 (US)
(74) Representative: Dr. Weitzel & Partner
(86) International application number: PCT/US2003/039893
(87) International publication number: WO 2004/060285

(56) References cited:
- WO-A-02/36364
- WO-A-02/41855
- FR-A1- 2 822 678
- US-A- 5 858 371
- US-A- 5 858 371
- US-A1- 2004 126 344
- DATABASE BIOSIS [Online] BIOSCIENCES INFORMATION SERVICE, PHILADELPHIA, PA, US; 2000, UPADHYE ANURADHA S ET AL: "Palynology of crude flower drugs from Pune market" XP002415799 Database accession no. PREV200100341370 & JOURNAL OF MEDICINAL AND AROMATIC PLANT SCIENCES, vol. 22-23, no. 4A-1A, 2000, pages 633-636,
- DATABASE CAPLUS [Online] CHEMICAL ABSTRACTS SERVICE, COLUMBUS, OHIO, US; XP002415800 retrieved from STN Database accession no. 1991:468450
- DATABASE CAPLUS [Online] CHEMICAL ABSTRACTS SERVICE, COLUMBUS, OHIO, US; XP002415801 retrieved from STN Database accession no. 1996:120043
- DATABASE WPI Week 200114 Derwent Publications Ltd., London, GB; AN 2001-123758 XP002415822 & CN 1 147 397 A (HU C) 16 April 1997 (1997-04-16)
- DATABASE WPI Week 200363 Derwent Publications Ltd., London, GB; AN 2003-666723 XP002415823 & JP 2003 113031 A (TS EARTH KK) 18 April 2003 (2003-04-18)

## Description

### BACKGROUND OF THE INVENTION

### 1. Field of the Invention

The present invention relates to topical compositions having an active ingredient naturally or synthetically derived from a plant or plant material. More particularly, the present invention relates to topical compositions that improve the appearance of skin, lips. Most particularly, the present invention relates to biologically active extracts for improving the aesthetic appearance of the skin, lips.

### 2. Description of the Related Art

Consumers constantly seek to improve the appearance of their skin, lips. There is a need for products that effectively reduce pigmentation in the skin, lips. Common applications for such products include, for example, bleaching hyperpigmented hair, skin, lips and/or nails; reducing age spots; evening or optimizing skin discoloration; improving the appearance of dark circles under the eyes; treating melasma, cholasma, freckles, after-burn scars, and post-injury hyperpigmentation; bleaching hair on the Scalp; legs, face, and other areas where bleaching and color reduction are desired; and bleaching nail stains.

Skin and lip pigmentation is determined by the level of melanin present in the epidermis. Three different types of melanin are present in the epidermis: DHI-melanin, DHICA-melanin and pheomelanin. The different types of melanin vary in color or shade. DHI-melanin is the darkest, and is blackish in color. DHICA-melanin is brownish in color. Pheomelanin is the lightest, and is reddish in color.

Melanin is synthesized in specialized organelles called melanosomes within pigment-producing cells (melanocytes). Melanocytes respond to stimuli to regulate melanin synthesis.

Many substances have been applied to the skin. Such substances include hydroquinone, kojic acid, licorice and/or its derivatives, ascorbic acid and/or its derivatives, arbutin, bearberry, *Glycyrrhiza glabra* and its derivatives, *Chlorella vulgaris* extract, perilla extract, and coconut fruit extract. Perilla extract is disclosed as a whitening agent in U.S. Patent No. 5,980,904 and Japanese Publications Nos. 07025742, 07187989, 10265322, 2001163759, and 2001181173. Coconut fruit extract is disclosed as a whitening agent in Japanese Patent No. 2896815B2. An extract of the spongy mass of coconut tissue is employed in a tanning sunscreen composition in U.S. Patent No. 5,756,099.

Active ingredients derived from plants and plant seeds have been employed in topical compositions for a myriad of medicinal, therapeutic and cosmetic purposes. Such active ingredients can be obtained from various parts of a'plant such as seeds, needles, leaves, roots, bark, cones, stems, rhizomes, callus cells, protoplasts, organs and organ systems, and meristems. Such active ingredients are incorporated in such compositions in a variety of forms. Such forms include a pure or semi-pure component, a solid or liquid extract or derivative, or a solid plant matter. Plant matter may be incorporated in a variety of subforms such as whole, minced, ground or crushed.

Extracts of *Azadirachta indica,* the neem tree, as well as other plants in the family *Meliaceae*, are known to have insecticidal activity. Azadirachtin, a major active ingredient of many of these extracts, is a liminoid of the tetranortriterpenoid type useful in commercial insecticides. Tetranortriterpenoids have been shown to be a potent insect growth regulator and feeding deterrent. Methods for producing azadirachtin concentrates from neem seed materials are known in the art. U.S. Patent No. 5,698,423 to Holowach-Keller et al. is directed to a method for producing azadiractin by cell culture of *Azadiracta india.*

Extracts of *Glycyrrhiza glabra linn.* are derived from the herb, which grows perennially in subtropical and warm temperate regions. *Glycyrrhiza glabra linn.,* commonly known as licorice, has been used in food sweetening. The root extract contains glycyrrhizic acid and glycyrrhetinic acid. The glycyrrhizic acid is known to have an anti-inflammatory effect. The extract of the licorice root and glycyrrhetinic acid have been shown to have desoxycorticosterone and ACTH-like effects. It has been used as a demulcent and mild expectorant. In vitro studies have shown the antiviral properties of both glycyrrhetinic acid and glycyrrhizin (See Badam, "In Vitro Studies on the Effect of Glycyrrhizin from Glycyrrhiza glabra linn. on Some RNA and DNA Viruses," Ind. J. Pharma., 26, 194-199 (1994)). The extracts of *Glycyzrhiza glabra linn.* can be extracted by a method disclosed in U.S. Patent No. 6,248,309 to Iyer, et al.

Extracts of *Morinda* citrifolia are derived from the Indian Mulberry plant. *Morinda citrifolia* has been used in compositions for reducing oxysterol buildup in the blood and normalizing cholesterol and blood pressure in mammals as set forth in U.S. Patent No. 6,387,370 to Yegorova. A method of extracting and purifying an essential oil product of *Morinda citrifolia* is disclosed in U.S. Patent No. 6,417,157 to Wadsworth et al.

Extracts of tomato glycolipid are derived from tomato fruit. Methods of extracting and synthesizing tomato glycolipids are disclosed in U.S. Patent No. 4,745,186 to Mudd et al.

Extracts of *Butea frondosa,* also known as Butea *monosperma*, are derived from an East Indian deciduous tree. *Butea frondosa* has been used as an astringent and in treating diarrhea, dysentery, and pyrosis. Use of *Butea frondosa* for its ocular anti-inflammatory activity has recently been tested (See Mengi, "Evaluation of Ocular Anti-Inflammatory Activity of Butea frondosa, " Ind. J. Pharma. 27, 116-119 (1995)).

*Extracts* of *Naringi crenulata,* also known as Limonia *crenulata*, are derived from a small tree indigenous to East India.

*Stenoloma chusana* is a perennial herb found in southeast Asia. Extracts from this plant are known to have uses in treating colds, influenza, bronchitis, burns, cuts, and skin sores (See A Barefoot Doctor's Manual, Running Press, Philadelphia, PA, p.638).

Heretofore, these extracts have not been used as an active ingredient in a composition for the purpose of lightening skin, lips, hair or nails.

It would be desirable to have compositions that employ new biological extracts that provide an improved aesthetic appearance to the skin and to the lips. It would further be desirable to have compositions that require minimal concentrations of the biological material.

### SUMMARY OF THE INVENTION

It is an object of the present invention to provide cosmetic compositions that improve the aesthetic appearance of skin and of the lips especially remediating the effects of aging.

It is another object of the present invention to provide compositions having an active plant extract, preferably an active biological plant extract.

It is yet another object of the present invention to provide such compositions for improving the appearance, in which the compositions having an effective amount of an active plant extract.

It is still another object of the present invention to provide such compositions for improving the appearance, in which such compositions are suitable for topical application to the skin or to the lips.

It is a still further object of the present invention to provide methods that include topically applying such compositions to skin or lips.

### DETAILED DESCRIPTION OF THE INVENTION

The present invention provides compositions for improving the appearance of skin, or lips. The compositions are topical compositions for application to the skin or to the lips. The compositions of the present invention also provide an anti-inflammatory benefit when applied to skin or lips, so that the appearance of skin is improved.

The compositions have one or more of the following extracts, as an active biological plant extract or ingredient(s) or in an active amount: *Butea* frondosa, *Naringi crenulata, Stenoloma chusana,* or any combinations thereof. It has been unexpectedly found that these active extracts improve the aesthetic appearance of skin or lips and, in particular, enhance kerakinocyte proliferation in skin or lips and/or enhance thickness of skin or lips. More particularly, these active extracts have been unexpectedly shown to decrease melanin production and decrease hypermelanacytic states.

It has now been also found that the addition of at least one of these active extracts, namely *Butea frondosa*, *Naringi crenulata*, *Stenoloma* chusana, or any combinations thereof, to at least one of the following other or additional extracts, namely *Azadirachta indica, Glycyrrhiza glabra linn*., *Morinda citrifolia,* tomato glycolipid, or any combinations thereof, can reduce melanin pigmentation of skin and of lips.

The benefits of the invention include bleaching hyperpigmented hair skin, or lips, reducing age spots; evening or optimizing skin discoloration; improving the appearance of dark circles under the eyes; treating melasma, cholasma, freckles, after-burn scars, and post-injury hyperpigmentation; .

Most generally, the present invention also provides topical compositions containing an effective amount of one or more of the active ingredients of the present invention for application to the skin to improve the aesthetic appearance of skin. These benefits are manifest in any of the following: reduction in pore size; improvement in skin tone, radiance, clarity and/or tautness; promotion of anti-oxidant activity; improvement in skin firmness, plumpness, suppleness, and/or softness; improvement in procollagen and/or collagen production; improvement in skin texture and/or promotion of retexturization; improvement in skin barrier repair and/or function; improvement in appearance of skin contours; restoration of skin luster and/or brightness; replenishment of essential nutrients and/or constituents in the skin decreased by aging and/or menopause; improvement in communication among skin cells; increase in cell proliferation and/or'multiplication; increase in skin cell metabolism decreased by aging and/or menopause; improvement in skin moisturization; promotion and/or acceleration of cell turnover; enhancement of skin thickness; reducing skin sensitivity; increase in skin elasticity and/or resiliency; and enhancement of exfoliation, with or without the use of alpha or beta hydroxy acids, keto acids or other exfoliants.

Collaterally, the present invention also provides for topical compositions for application to the skin that provide an anti-aging benefit. The compositions have an effective amount of one or more active ingredients of the present invention, which, when applied to human skin, prevent, treat and/or ameliorate the various signs of aging at the area or portion of skin to which they are applied. In particular, the present invention provides compositions and methods for treating skin to prevent, inhibit, reduce and/or ameliorate the signs of dermatological aging due to, for example, chronological aging, hormonal aging, and/or photoaging. Such signs of aging include, but are not limited to skin fragility; loss of collagen and/or elastin; estrogen imbalance in skin; skin atrophy; appearance and/or depth of lines and/or wrinkles, including fine lines; skin discoloration, including dark eye circles; skin sagging; skin fatigue and/or stress, e.g., skin breakout due to environmental stress, such as pollution and/or temperature changes; skin dryness; skin flakiness; cellular aging; loss of skin tone, elasticity and/or luster; loss of skin firmness; poor skin texture; loss cf skin elasticity and/or resiliency; and thin skin.

In its broadest aspects, the present invention is not limited by any particular characterization of the physiological and/or chemical effects of the active extract or agents.

Accordingly, the method of the present invention provides that an effective amount from about 0.001 wt% to about 20 wt% by weight of the composition, is effective in the treatment of one or more of the following: reducing or preventing loss of collagen; improving skin firmness/ plumpness; improving skin texture; decreasing/preventing lines and wrinkles; improving skin tone; enhancing skin thickness; decreasing pore size; reducing skin discoloration; reducing acne; reducing psoriasis; reducing skin sensitivity; and reducing warts.

Cosmetic, dermatological and pharmaceutical products commonly have an active agent or agents that produce skin irritation. Examples of active agents having skin irritation as a side effect include, but are not limited to, hydroxylated acids and their derivatives, α-hydroxy acids (i,e., lactic, glycolic, citric, malic, tartaric, mandelic, gluconic, methyl lactic, phenyl lactic, atrolactic, glyceric, benzilic, z-hydroxyheptanoic, z-hydroxyoctanoic and any combinations thereof), β-hydroxy acids (i.e., salicylic, 5-n-octanoylsalicylic and other derivatives of salicylic), retinoids (retinoic acid and its derivatives; retinol and its esters); anthralins (i.e., dioxyanthranol), anthranoids, peroxides (i.e., benzoyl peroxide), minoxidil, lithium salts, antimetabolites, vitamin D and its derivatives, hair dyes or colorants (i.e., para-phenylenediamine and its derivatives; aminophenols), alcoholic perfuming solutions (i.e., perfumes; toilet waters; aftershaves; deodorants), antiperspirant agents (i.e., some aluminum, salts), depilatory or hair permanent active agents (i.e., thiols), depigmentating agents (i.e., hydroquinone), and some insecticide active agents. If topical products had anti-irritant protection, it would be possible to increase the amount of the normal irritant active agent (i.e., AHA or BHA) in the product without producing unpleasant skin irritation or irritation side effects. The use of the present compositions makes it possible to improve the efficacy of cosmetic, dermatolagical or pharmaceutical products by increasing the concentration or amount of cosmetic, dermatological, or pharmaceutical active agent as compared to the amount or concentration of such agent normally used.

In a preferred embodiment of the present invention, the composition, which improves the appearance of skin and lips has an effective amount of at least one of the following active extracts: *Butea frondosa, Naringi crenulata*, *Stenoloma chusana*, or any combinations thereof. An effective amount means that the one or more active extracts are present at about 0.001 percentage by weight (wt%) to about 20 wt% based on the total weight of the composition. The one or more active extracts are present preferably at about 0.05 wt% to about 10 wt%, and more preferably at about 0.5 wt% to about 5 wt%, based on the total weight of the composition. Most preferably, the one or more active extracts are present in an amount about 1 wt% or less based on the total weight of the composition.

As stated above, in another preferred embodiment of the present invention, the composition has an effective amount of at least one of the active extracts, and one or more of the following other or additional extracts: Azadirachta *indica, Glycyrrhiza glabra linn*., *Morinda citrifolia,* tomato glycolipid, or any combinations thereof, to synergistically enhance the activity of the composition.

The one or more of the additional extracts are present in an amount about 0.C5 wt% to about 10 wt% based on the total weight of the composition. Preferably, the one or more additional extracts in an amount about 0.5 wt% to about 5 wt% based on the total weight of the composition. Most preferably, the one or more additional extracts are present in an amount equal to or less than 1 wt% based on the total weight of the composition.

When combined with one or more of the additional extracts, the active extracts are present in the present compositions in an amount about 0.001 wt% to about 10 wt%, and the one or more additional extracts in an amount about 0.05 wt% to about 10 wt%, based on the total weight of the composition. Preferably, the active extracts are present in an amount about 0.05 wt% to about 10 wt%, and the one or more additional extracts in an amount about 0.5 wt% to about 5 wt% based on the total weight of the composition. Most preferably, the one or more active extracts are present in an amount equal to or less than about 1 wt%, and the one or more additional extracts are present in an amount equal to or less than 1 wt%, based on the total weight of the composition.

The compositions of the present invention can be used with a carrier or vehicle. The vehicle can be altered to be appropriate for the specific use cf the composition without altering the beneficial lightening effects achieved by the use of the one or more active extracts set forth above. The vehicles that can be used in compositions of the present invention include, but are not limited to, water; vegetable oils; esters such as octyl palmitate, isopropyl myristate and isopropyl palmitate; ethers such as dicapryl ether and dimethyl isosorbide; alcohols such as ethanol and isopropanol; fatty alcohols such as cetyl alcohol, stearyl alcohol and behenyl alcohol; isoparaffins such as isooctane, isododecane and isohexadecane; silicone oils such as dimethicones and polysiloxanes; hydrocarbon oils such as mineral oil, petrolatum, isoeicosane and polyisobutene; polyols such as propylene glycol, glycerin, butylene glycol, pentylene glycol and hexylene glycol; or any combinations thereof.

Preferably, the vehicle is present in an amount about 50 wt% to about 99.8 wt% based on the total weight of the composition. More preferably, the vehicle is present in an amount about 80 wt% to about 99.5 wt% based on the total weight of the composition.

The present compositions may also include one or more of the following ingredients: anesthetic, antiallergenic, antifungal, antimicrobial, anti-inflammatory agent, antioxidant, antiseptic, chelating agent, colorant, depigmenting agent, emollient, emulsifier, exfollient, film former, fragrance, humectant, insect repellent, lubricant, moisturizer, pharmaceutical agent, photostabilizing agent, preservative, skin protectant, skin penetration enhancer, sunscreen, stabilizer, surfactant, thickener, viscosity modifier, vitamin, or any combinations thereof. Preferably, these ingredients are present in an amount about 0.001 wt% to about 10 wt% based on the total weight of the composition.

The present compositions may also include skin whiteners. Some examples of such suitable skin whiteners include, but are not limited to, one or more of the following: ascorbyl glucoside, vitamin C, retinol and/or its derivatives, arbutin, bearberry extract, rumex crispus extract, milk proteins including hydrolyzed milk proteins, N,N,S-tris(carboxymethyl)cysteamine, oleanolic acids, perilla oils, placenta extract, *saxifragia* sarmentosa, perilla extract, juniperic acid, TDPA, *ligusticum chiangxiong hort.,* asmunda *japonica thunb. , stellaria medica* (L.) *cyr*., *sedum sarmentosum bunge*, *ligusticum lucidum Ait*., *ilex purpurea hassk,* emblica, apigenin, ascorbyl palmitol, carruba polyphenols, hesperitin, hydroquinone, inabata polyphenol, isoliquirtigenin, kaempherol-7-neohesperidose, L-mimosine, luteolin, oil-soluble licorice extract P-T(40), oxa acid, phenyl isothiocyanate, cococin, silymarin, T4CA, teterahydro curcumin, unitrienol, ursolic-oleanolic acid, UVA/URSI, or any combinations thereof.

The compositions of the present invention can be formulated in any suitable product form. Such product forms include, but are not limited to, aerosol spray, cream, dispersion, emulsion, foam, gel, liquid, lotion, mousse, ointment, patch, pomade, powder, pump spray, solid, solution, stick, and towelette.

The present compositions can be formulated to deliver a consistent level of an active ingredient, or blend of ingredients, so that a desired cosmetic effect is achieved.

The following are examples of the present invention.

### EXAMPLE 1

Melanosome Uptake Assay *(Stenolama Chusana* and *Naringi Crenulata*) was done as follows. Confluent cultures of B16 melanocytes produce moderate levels of melanosomes. However, to induce elevated melanosome production in this cell line, semi-confluent (60%) cultures of B16 cells were treated for approximately thirty-six (36) hours with normal growth medium supplemented with 10 mM ammonium chloride (final conc.). The medium was then aspirated and the hypermelanotic cells were washed (2 x 2 ml) with distilled water to provide a hypotonic stress to the cells. An aliquot (2 ml) of a hypotonic lysis solution (0.02% NP-40 in water) was added to each plate and the plates were incubated for approximately five (5) minutes at room temperature. Following verification of cell lysis using light microscopy, the cellular material from three (3) culture plates were pooled in a 15 ml conical tube and centrifuged (200 x g) for 5 minutes to remove cellular debris. The resulting supernatant containing melanosomes was transferred to a clean 15 ml conical tube and centrifuged (850 x g) for twenty (20) minutes. The resulting pellet containing the isolated melanosomes were resuspended in 1 ml of Phosphate Buffered Saline (PBS) and stored at 4°C until used.

The treatment of keratinocytes with melanosomes was measured as follows. The normal human epidermal keratinocytes (NHEKs) (available from Clonetics, Inc.) were plated in the wells of 24-well plates at a density of 200,000 cells/well. Approximately twenty-four (24) hours later, the growth medium was replaced with 1 ml of the appropriate growth medium (i.e., DMEM/KGM-2) containing the melanosome preparation with or without additional treatment conditions. The cells were treated with different concentrations of perilla leaf extract (powder form or aqueous form). The cells were then returned to the incubator for approximately one and one-half (1.5) hours. For these studies, each well of keratinocytes was treated with the amount of melanosomes isolated from a single plate of B16 cells.

In some experiments, the 24-well plates of treated keratinocytes were centrifuged for 15 minutes at 1,000 rpm to facilitate the deposition of the melanosomes onto the surface membranes of the keratinocytes. The plates were then returned to the incubator for 1.25 hours.

For the analysis of melanosome uptake, the cells in each well of keratinocytes were rinsed (3 x 1 ml) with PBS, removed from the plate using trypsin/EDTA, and washed with PBS. To analyze the uptake of melanosomes by the keratinocytes, the internalized melanin was extracted from the cells according to a modified method of Bessou-Touya, S., et al. (Chimeric human epidermal reconstructs to study the role of melanocytes and keratinocytes in pigmentation and photoprotection. J. Invest. Dermatol., 111:1103-1108, 1998) and quantified spectrophotometrically by determining the melanin-specific absorbance at 405 nm.

The melanocytes synthesize melanin and deposits onto melanosomes. Visual manifestation of skin color is due to presence of melanin/melanosomes in keratinocytes. Melanosomes are taken up by keratinocytes and the rate of uptake, retention and processing of melanosomes in the keratinocytes is a key determinant of skin color. The internalized melanin value reflects the amount of melanin/melanosome uptake and retention by the keratinocytes. Thus, the lower internalized melanin values, particularly internalized melanin values that are less than the control with melanin, indicate that melanin uptake by the keratinocytes has been inhibited.

The results are as follows.At 0.5% volume/volume, *Stenolama Chusana* showed a 44% decrease in melanosome uptake as compared to the positive control. At 0.5% volume/volume, *Naringi Crenulata* showed a 39% decrease in melanosome uptake as compared to the positive control.

### EXAMPLE 2

For B16 assay *(Naringi Crenulata),* the actives were tested in monolayer cell culture of B16 mouse melanoma cells. These cells constitutively produce melanin and are a model system for monitoring the inhibition of melanin synthesis. The cells were seeded into 96-well plates at 5 x 10³ cells/well and culture attached for twenty-four (24) hours. The media were then replaced with fresh media containing plant extracts. Each active was applied to six (6) wells of B16 cells on 96-well plates to allow statistical analysis. The cells were dosed with medium alone as the negative control, or the test article for seven (7) days. The plates were read at 540nM to detect melanin formation. An increase in absorbance at 540 nM reflects a higher melanin content in the well.

At 0.05% weight/volume, *Naringi Crenulata* showed a 61% decrease in pigmentation as compared to the positive control

### EXAMPLE 3

For ¹⁴C-Dopa incorporation (*Butea Frondosa),* melanogenic activity was measured by measuring the radioactive melanin formed as ¹⁹C DOPA is converted to the acid insoluble melanin biopolymer in B16F10 melanoma cells. Cells were seeded into 24-well plates at a density of 2 x 10⁴ cells per well and cultured attached for forty-eight (48) hours. The media were then replaced with fresh media containing plant extracts and 0.2 µCi of ¹⁹C DOPA. The cells were further incubated for 24 hours. After incubation, media were discarded and the cells were rinsed with PBS, lysed by adding 0.125 ml of 1N NaOH and incubated at 37°C for 30 minutes, and then neutralized with 0.025 ml of 5N HCL. The resulting cell lysates were transferred into liquid scintillation vials and mixed with scintillation cocktail, and the radioactivity was determined by scintillation counter. A portion of the cell lysates was kept and the protein content was determined by Lowry method. The results were normalized to the amount of protein determined for each assay.

At 0.005% weight/volume, *Butea frondosa* showed a 22% decrease in ¹⁹C DOPA conversion as compared to the positive control.

### EXAMPLE 4

Various natural plant extracts of the present invention were evaluated for inhibition of the TNF-alpha protein in an in vitro studies as described below.

### TNF-alpha Inhibition Protocol

To test the efficacy of *Stenolama chusana Ching., Naringi* Crenulata, and *Butea frondosa Roxb.* for the inhibition of TNF-alpha production, an enzyme linked immunoassay (ELISA) commercially available from R&D Systems was employed.

This assay employed the quantitative sandwich enzyme immunoassay technique in which a monoclonal antibody specific for TNF-alpha has been pre-coated onto a microtiter plate. Culture supernatants from cells exposed to active materials were pipetted into separate wells. TNF-alpha in the supernatant was bound to the plate via the immobilized antibody. After several washes to remove unbound antibody, an enzyme-linked polyclonal antibody specific for TNF-alpha was added to each well. Following a wash to remove unbound antibody-enzyme reagent, a substrate solution was added to the wells. Color develops in proportion to the amount of TNF-alpha bound in the initial step. The results of the tests are provided in Table 1.

**Table I**

| Results of In Vitro Tests | |
|---|---|
| Plant Extract | TNF-alpha Inhibition |
| *Stenolama chusana Ching.* | +++ |
| *Naringi Crenulata* | + |
| *Butea frondosa Roxb*. | +++ |

| | |
|---|---|
| Legend: +, ++, +++, ++++ Significant inhibition (degree of inhibition quantified by number of plus signs) (0) No change | |

These in vitro studies show that the compositions of the present invention containing an extract of: *Stenolama chusana Ching., Naringi Crenulata, and Butea frondosa Roxb*. provide benefits to the skin by inhibiting TNF-alpha protein production, whereby the signs of subjective discomfort and/or irritation caused by cosmetic, pharmaceutical or dermatological products would be reduced, thus providing an aesthetic improvement in skin appearance.

### EXAMPLE 5

*Stenolama chusana ching.* and *Butea fondosa* were evaluated in various biopsy studies in which the natural plant extract as set forth below was incorporated into a cosmetically suitable vehicle and applied to the volar forearm of a participant in the study, at a dose of 2 mg/cm². The forearm area to which the extract preparation was applied was then covered with a semi-occlusive patch. This procedure was repeated for 3 weeks, 5 days per week. At the end of the treatment the sites were anesthetized with lidocaine and 2 mm punch biopsies were taken from the treated and one untreated control site. Biopsies were fixed in formalin, embedded in paraffin, sectioned, and stained for relevant endpoints.

For *Stenolama chusana ching.,* 6 of 9 panelists showed increase in keratinocyte proliferation (visualized by the antibody to KI67), and 5 of 9 panelists showed increase in viable epidermal thickness.

For *Butea frondosa* provided, 5 of 8 panelists showed increase in keratinocyte proliferation (visualized by the antibody to KI67), and 5 of 8 panelists showed increase in viable epidermal thickness.

It should be understood that the foregoing description is only illustrative of the present invention. Various alternatives and modifications can be devised by those skilled in the art without departing from the invention. Accordingly, the present invention is intended to embrace all such alternatives, modifications and variances which fall within the scope of the appended claims.

## Claims

1. Cosmetic use of a topical composition with at least one active extract selected from the group consisting of Naringi crenulata, Stenoloma chusana and Butea frondosa for enhancing keratinocyte proliferation in skin or lips and/or for enhancing thickness of skin or lips.

2. Cosmetic use of claim 1, wherein the cosmetic composition is further used to prevent loss of collagen and/or elastin in skin or lips.

3. Cosmetic use of one the preceding claims, wherein the cosmetic composition is further used to decrease and/or prevent lines and wrinkles.

4. Cosmetic use of one of the preceding claim, wherein the composition further comprises a vehicle, wherein the at least one extract is present in an amount 0.001 wt% to 20 wt% based on the total weight of the composition.

5. Cosmetic use of one of the preceding claims, wherein the composition further comprises a vehicle, wherein the at least one extract is present in an amount 0.05 wt% to 10 wt% based on the total weight of the composition.

6. Cosmetic use of one of the preceding claims, wherein the composition further comprises a vehicle, wherein the at least one extract is present in an amount of 0.5 wt% to 5 wt% based on the total weight of the composition.

7. Cosmetic use of one of the preceding claims, wherein the at least one extract is Naringi crenulata.

8. Cosmetic use of one of the preceding claims, wherein the at least one extract is Stenoloma chusana.

9. Cosmetic use of one of the preceding claims, wherein the at least one extract is Butea frondosa.

10. Cosmetic use of one of the preceding claims, further comprising at least one additional extract selected from the group consisting of Azadirachta Indica, Glycyrrhiza glabra Ilnn., Morinda citrifolla, and tomato glycolipid.

## Patentansprüche

1. Kosmetische Anwendung einer topischen Zusammensetzung mit mindestens einem wirksamen Auszug, der aus der Gruppe aus Naringl crenulata, Stenoloma chusana und Butea frondosa bestehend gewählt ist, zur Verbesserung der Wucherung von Keratinozyten in der Haut beziehungsweise den Lippen und/oder zur Verbesserung der Haut- beziehungsweise Lippendicke.

2. Kosmetische Anwendung nach Anspruch 1, wobei die kosmetische Zusammensetzung ferner genutzt wird, um den Verlust an Kollagen und/oder Elastin in der Haut beziehungsweise in den Lippen zu verhindern.

3. Kosmetische Anwendung nach einem der vorhergehenden Ansprüche, wobei die kosmetische Zusammensetzung ferner genutzt wird, um Linien und Runzeln zu verringern und/oder verhindern.

4. Kosmetische Anwendung nach einem der vorhergehenden Ansprüche, wobei die Zusammensetzung eine Trägersubstanz ferner enthält, wobei der mindestens eine Auszug, basiert auf dem Gesamtgewicht der Zusammensetzung, In einer Menge von 0,001 Gwt.% bis 20 Gwt.% vorhanden ist.

5. Kosmetische Anwendung nach einem der vorhergehenden Ansprüche, wobei die Zusammensetzung eine Trägersubstanz ferner enthält, wobei der mindestens eine Auszug, basiert auf dem Gesamtgewicht der Zusammensetzung, in einer Menge von 0,05 Gwt.% bis 10 Gwt.% vorhanden ist.

6. Kosmetische Anwendung nach einem der vorhergehenden Ansprüche, wobei die Zusammensetzung eine Trägersubstanz ferner enthält, wobei der mindestens eine Auszug, basiert auf dem Gesamtgewicht der Zusammensetrung, in einer Menge von 0,5 Gwt.% bis 5 Gwt.% vorhanden ist.

7. Kosmetische Anwendung nach einem der vorhergehenden Ansprüche, wobei der mindestens eine Auszug Naringi crenulata ist.

8. Kosmetische Anwendung nach einem der vorhergehenden Ansprüche, wobei der mindestens eine Auszug Stenoloma chusana ist.

9. Kosmetische Anwendung nach einem der vorhergehenden Ansprüche, wobei der mindestens eine Auszug Butea frondosa ist.

10. Kosmetische Anwendung nach einem der vorhergehenden Ansprüche, ferner umfassend mindestens einen weiteren Auszug, der sich aus Azadirachta Indica, Glycyrrhiza glabra Llnn., Morinda citrifolla und Glykolipid von Tomate zusammensetzenden Gruppe gewählt ist.

## Revendications

1. Utilisation cosmétique d'une composition topique comprenant au moins un extrait actif, choisi parmi le groupe consistant en Naringi crenulata, Stenoloma chusana et Butea frondosa, pour améliorer la prolifération de kératinocytes dans la peau ou dans les lèvres et/ou pour renforcer l'épaisseur de la peau ou des lèvres.

2. Utilisation cosmétique selon la revendication 1, dans laquelle la composition cosmétique permet en outre, de prévenir la perte de collagène et/ou d'élastine dans la peau ou dans les lèvres.

3. Utilisation cosmétique selon l'une quelconque des revendications précédentes, dans laquelle la composition cosmétique permet en outre de réduire et/ou de prévenir les lignes et les rides.

4. Utilisation cosmétique selon l'une quelconque des revendications précédentes, dans laquelle la composition comporte de plus un excipient tandis que ledit au moins un extrait, sur la base du poids total de la composition, est présent dans une quantité de 0,001 % en poids à 20 % en poids.

5. Utilisation cosmétique selon l'une quelconque des revendications précédentes, dans laquelle la composition comporte de plus un excipient tandis que ledit au moins un extrait, sur la base du poids total de la composition, est présent dans une quantité de 0,05 % en poids à 10 % en poids.

6. Utilisation cosmétique selon l'une quelconque des revendications précédentes, dans laquelle la composition comporte de plus un excipient tandis que ledit au moins un extrait, sur la base du poids total de la composition, est présent dans une quantité de 0,5 % en poids à 5 % en poids.

7. Utilisation cosmétique selon l'une quelconque des revendications précédentes, tandis que ledit au moins un extrait consiste en Naringi crenulata.

8. Utilisation cosmétique selon l'une quelconque des revendications précédentes, tandis que ledit au moins un extrait consiste en Stenoloma chusana.

9. Utilisation cosmétique selon l'une quelconque des revendications précédentes, tandis que ledit au moins un extrait consiste en Butea frondosa.

10. Utilisation cosmétique selon l'une quelconque des revendications précédentes, comportant de plus au moins un autre extrait choisi parmi le groupe consistant en Azadirachta Indica, Glycyrrhlza glabra Linn., Morinda citrifolla et glycolipide de tomate.
